# EUROPEAN PATENT APPLICATION

(11) **EP 2 452 669 A1**
(43) Date of publication of application: **16.05.2012**
(21) Application number: 10380137.9
(22) Date of filing: 29.10.2010
(51) Int. Cl.: A61K 9/00, A61K 31/5575, A61P 27/02, A61P 27/06

(54) **Ophthalmic composition**

(71) Applicant: Omnivision GmbH, 82178 Puchheim (DE)
(72) Inventor: Rizzo Tamaro, Adriana, 08191 Rubi-Barcelona (ES)
(74) Representative: Blodig, Wolfgang

(57) **Abstract**

The present invention relates to an aqueous ophthalmic composition comprising
(i) a prostaglandin; and
(ii) one or more anionic surfactants,
characterized in that
the concentration of anionic surfactant in the ophthalmic composition is from 0.001 to 1.0 g/L.

## Description

### FIELD OF THE INVENTION

The present invention provides an aqueous ophthalmic composition containing a prostaglandin such as a prostaglandin FP receptor agonist as active ingredient. The invention also provides an ophthalmic composition, such as an eye drop formulation, for use in the treatment of glaucoma or intraocular hypertension. Also disclosed is a method of preventing or treating glaucoma or intraocular hypertension. The ophthalmic composition of the invention is free of potentially irritating preservatives such as benzalkonium chloride.

### BACKGROUND OF THE INVENTION

Prostaglandins are well known active ingredients administered to humans or animals via the topical route in the form of ophthalmic solutions for the treatment of glaucoma or intraocular hypertension. The usual dosage for these formulations is 1 drop per day in both eyes. Prostaglandins may also be used in combination with a second anti-glaucoma agent such as a beta-blocker, a carbonic anhydrase inhibitor or an alpha-adrenergic agonist.

The prime disadvantage of prostaglandins is that they have a low water solubility so that special measures have to be taken to stably solubilise the prostaglandin in water. Another constraint on making the formulation is the requirement to provide an ophthalmic solution which is chemically stable over usual storage times. Further, the ophthalmic solution should be stable against adsorbtion of the prostaglandin to the packaging material in which it is stored, in particular relative to packaging in low-density polyethylene (LDPE) material.

As described in US 2010/0210720, currently most prostaglandin based ophthalmic solutions on the market contain a preservative agent which, besides having antimicrobial properties, also solubilises the active ingredient and partly stabilizes it. An example of such a solution is the product marketed under the trade name of Xalatan(R) by Pfizer, containing latanoprost and 0.02% by weight benzalkonium chloride (BAC). Despite the presence of BAC, this ophthalmic solution is not stable at ambient temperature and must be stored in the cold at a temperature of about 5°C.

However, the use of antimicrobial preservatives such as BAC in ophthalmology for long-term treatment is problematic, since it attacks the cornea and causes side-effects such as macular edema and keratoconjunctive disorders. Therefore, it is desired to eliminate preservatives from ophthalmic compositions.

WO 97/29752 discloses the use of a non-ionic agent such as Cremophor™ as partial replacement of BAC. Polysorbate 80 has also been used in ophthalmic solutions as a partial substitute for BAC, as is the case for the product marketed under the trade name Rescula, containing unoprostone with a mixture of BAC and polysorbate 80 forming 0.015% by weight of the solution.

EP 1 321 144 and EP 1 666 043 describe the use of polysorbate 80 to slow down adsorption of a specific prostaglandin, tafluprost, to polyethylene (PE), low density polyethylene (LDPE), polypropylene (PP), polyethylene terephthalate (PET) or a mixtures thereof to the packaging material. These documents state BAC as possible additive for preservation.

US2004/0082660 describes an ophthalmic solution without BAC containing a mixture of latanoprost and polysorbate 80. Experimental data are given on the homogeneity of the solutions in the presence of 0.2 g/I polysorbate 80 thirty minutes after 7 hours of agitation. Thus, this document is concerned with the capacity of polysorbate 80 to solubilise latanoprost in 7 hours.

US 2010/0210720 describes an ophthalmic solution without antimicrobial preservatives. The formulations of the examples include at least one prostaglandin in an amount of 0.005 % and, as a solubilising agent, 0.5 % polyoxyl-15-hydroxystearate (Solutol HS15). Thus, the surfactant is used in a huge excess over the active ingredient.

JP 2009-40749-A relates to the problem of providing a stable latanoprost formulation and describes ophthalmic solutions containing the stearic acid ester monostearic acid polyethylene glycol as an inhibitor of adsorption of latanoprost to resin containers. However, the formulations of this document contain BAC for preservation.

Most prostaglandins used in eye drops for the treatment of glaucoma or ocular hypertension are pro-drugs having the prostaglandin carboxylic acid group protected by ester or amide moieties. Pro-drug formation has the purpose of improving penetration of the compounds into tissue. The active drug which is the free carboxylic acid is formed after application to the eye from the pro-drug by hydrolysis. Among the processes that reduce the amount of the prostaglandin active ingredient in an aqueous solution over time is (apart from adsorbtion to container walls) hydrolysis of the carboxyl-protective group, such as the isopropyl ester group of latanoprost. Although the decomposition product is the substance that acts on the target receptor (the prostaglandin FP receptor), decomposition by hydrolysis in the drug formulation upon storage influences pharmacokinetics, and thus makes the pharmacokinetic properties of the drug time-dependent. Naturally, it is desired that a drug formulation has stable pharmacokinetic properties and concentrations over the usual storage times. In order to account for loss by hydrolysis, excess amounts of pro-drug are sometimes used in order to account for such losses, see EP 1 011 728 B1 of Alcon Manufacturing, Ltd. that uses a 5% excess of the pro-drug in examples 1 and 2. Thus, it is desired to provide stable aqueous prostaglandin formulations wherein hydrolysis can be suppressed during manufacture of the formulations and possibly also during storage.

However, due to the high hydrophobicity of the prostaglandins used in eye drops, preparation of clear aqueous formulations is difficult. In order to dissolve the prostaglandin in the carrier solution, quite harsh conditions such as use of elevated temperatures or use of ultrasound are frequently used. Alternatively, solubilisation takes a long time. For example, heating to 80°C is used in the examples of EP 2 123 278 A1; sonication and stirring overnight is used in US 5,849,792; agitation over 7 hours is done in the example of EP 1 553 953 B1. These methods are, however, disadvantageous from the point of view of avoiding decomposition by hydrolysis. These problems are further reinforced, if established solubilisers such as BAC are to be avoided, and if the content of surfactants that have the potential to influence the surface properties of the cornea is to be reduced as far as possible.

In spite of extensive research over the years, the prior art still does not provide an aqueous ophthalmic composition for the treatment of glaucoma or ocular hypertension, that can quickly and stably solubilise prostaglandins using low amounts of surfactants as solubilisers even in the absence of BAC or similar kationic antimicrobial agents and in the presence of low amounts of other surfactants as solubilising agents.

### SUMMARY OF THE INVENTION

Departing from the prior art, it is a problem of the present invention to provide a storage stable ophthalmic composition for the treatment of glaucoma or ocular hypertension, wherein the ophthalmic composition is free of antimicrobial agents such as BAC and allows fast and stable solubilisation of prostaglandin anti-glaucoma agents. Thus, the invention provides the an aqueous ophthalmic composition comprising
(i) a prostaglandin; and
(ii) one or more anionic surfactants,
   characterized in that
the concentration of anionic surfactant in the ophthalmic composition is from 0.001 to 3.0 g/L, preferably 0.005 to 1.0 g/L, more preferably 0.005 to 0.5 g/L, and even more preferably 0.005 to 0.1 g/L.

The invention also provides the method according to claim 10, a method of treatment according to claim 12, and a packaged product according to claim 13. The invention also provides the ophthalmic composition for use in the treatment of glaucoma or intraocular hypertension.

The inventor has surprisingly found that anionic surfactants have a high solubilising power for prostaglandin anti-glaucoma agents. As a result, the prostaglandins can be solubilised in water or aqueous solutions in a short period of time even under mild conditions such as cooling to below 10 or even below 5°C, which suppresses hydrolysis of the prostaglandin pro-drugs. Moreover, storage stable prostaglandin ophthalmic compositions, notably clear solutions thereof for eye drop formulations, containing very small concentrations of the anionic surfactant can be prepared, thus avoiding irritating effects of the surfactant on the eyes. It is even possible to use anionic surfactant concentrations that are below those of the prostaglandin (in terms of weight per volume of formulation). The compositions of the invention are prostaglandin eye drop formulations free of BAC or other cationic antimicrobial agents.

### DETAILED DESCRIPTION OF THE INVENTION

The present invention relates to an aqueous ophthalmic composition. The aqueous ophthalmic composition is a clear aqueous solution. The aqueous ophthalmic composition preferably does not comprise particulate matter and is preferably neither a suspension nor a gel.

The aqueous ophthalmic composition of the present invention comprises, in addition to water, a prostaglandin and one or more anionic surfactants.

The prostaglandin is preferably an analogue of prostaglandin F_{2α} (Dinoprost). An analogue of prostaglandin F_{2α} is a compound which comprises a 1,2-disubstituted-3,5-dihydroxycyclopentane ring having the stereochemistry corresponding to prostaglandin F_{2α}. In particular, the analogue of prostaglandin comprises a (1*R*,2*R*,3*R*,5*S*)-3,5-dihydroxycyclopentane ring having substituents at the 1- position and at the 2-position. Preferably, the 1-position is substituted with a hydrocarbon chain which terminates with a linear or branched C₁-C₆ alkyl ester moiety or a linear or branched C₁-C₆ alkyl amide moiety, and the 2-position of the cyclopentyl ring is substituted with a (CH₂)₂ moiety or a *trans*-CH=CH moiety, to which an optionally fluorinated hydrocarbon chain comprising at least one hydroxyl, ketone or ether moiety is attached.

In one embodiment, the prostaglandin is of the formula (1): 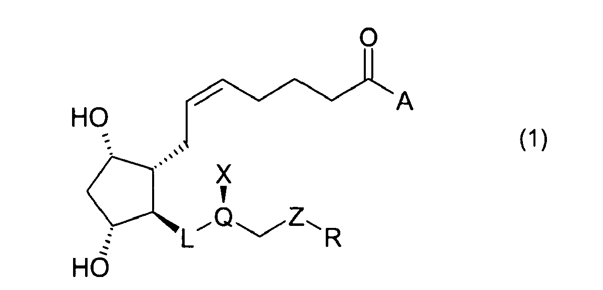 wherein
- A: is a linear or branched C₁-C₆ alkoxy moiety or a linear or branched mono- or di-C₁-C₆ alkylamino moiety;
- L: is a (CH₂)₂ moiety or a *trans*-CH=CH moiety;
- Q: is C, a CH moiety, or a CF moiety, with the proviso that when
(a) Q is C, X is O and the Q-X bond is a double bond,
(b) Q is CH, X is OH, and
(c) Q is CF, X is F;
- R: is a linear, branched or cyclic C₃-C₈ alkyl moiety, a phenyl moiety, or a phenyl moiety which is substituted with
(i) a linear or branched C₁-C₃ alkyl moiety,
(ii) a halogen, or
(iii) a linear or branched C₁-C₃ haloalkyl moiety; and
- Z: is O or a CH₂ moiety.

In a preferred embodiment, the prostaglandin is of the formula (1), wherein
- A: is a linear or branched C₁-C₆ alkoxy moiety;
- L: is a (CH₂)₂ moiety or a *trans*-CH=CH moiety;
- Q: is C, a CH moiety, or a CF moiety, with the proviso that when
(a) Q is C, X is O and the Q-X bond is a double bond,
(b) Q is CH, X is OH, and
(c) Q is CF, X is F;
- R: is a linear, branched or cyclic C₃-C₈ alkyl moiety, a phenyl moiety, or a phenyl moiety which is substituted with
(i) a linear or branched C₁-C₃ alkyl moiety,
(ii) a halogen, or
(iii) a linear or branched C₁-C₃ haloalkyl moiety; and
- Z: is O or a CH₂ moiety.

In a more preferred embodiment, the prostaglandin is of the formula (1), wherein
- A: is a linear or branched C₁-C₆ alkoxy moiety;
- L: is a (CH₂)₂ moiety or a *trans-*CH=CH moiety;
- Q: is C, a CH moiety, or a CF moiety, with the proviso that when
(a) Q is C, X is O and the Q-X bond is a double bond,
(b) Q is CH, X is OH, and
(c) Q is CF, X is F;
- R: is a propyl or pentyl moiety, a phenyl moiety or a 3-(trifluoromethyl)phenyl moiety; and
- Z: is O or a CH₂ moiety.

Preferably, A is an isopropoxy moiety or ethylamino moiety, such that the hydrocarbon chain which is substituted at the 1-position of the cyclopentyl ring terminates with an isopropyl ester moiety or an ethylamide moiety, respectively. More preferably, A is an isopropoxy moiety.

Yet more preferably, the prostaglandin is selected from latanoprost (CAS 130209-82-4), bimatoprost (CAS 155206-00-1), travoprost (CAS 157283-68-6), tafluprost (CAS 209860-87-7) and unoprostone isopropyl (CAS 120373-24-2). Most preferably, the prostaglandin is latanoprost.

The analogue of prostaglandin F_{2α} is preferably a compound which binds to a receptor for prostaglandin F_{2α}. In one embodiment, the prostaglandin is a prostaglandin FP receptor agonist. A prostaglandin FP receptor agonist is preferably a compound which binds to a receptor for prostaglandin F_{2α} and triggers a response therefrom. Such a response preferably involves, *inter alia,* modulation of intraocular pressure. Modulation of intraocular pressure may involve reducing said intraocular pressure through increasing the uveoscleral outflow of the aqueous humor.

The concentration of prostaglandin in the aqueous ophthalmic composition is preferably from 0.001 to 0.1 g/L, more preferably from 0.01 to 0.07 g/L, even more preferably from 0.03 to 0.06 g/L.

The one or more anionic surfactants are selected from surfactants which comprise a hydrophobic organic moiety and a hydrophilic moiety. The hydrophilic moiety is an anion, wherein the anion is selected from sulfate, sulfonate, phosphate, phosphonate, sarcosinate and carboxylate moieties. Thus, the one or more anionic surfactants may comprise organosulfate surfactants selected from C₁₀-C₂₂ alkylsulfates, C₁₀-C₂₂ alkyl(oligooxyalkylene)sulfates, and C₁₀-C₂₂ alkylbenzyl(oligoxyalkylene)sulfates; organosulfonate surfactants selected from C₁₀-C₂₂ alkylsulfonates, C₁₀-C₂₂ alkyl(oligooxyalkylene)sulfonates, C₁₀-C₂₂ acyl(oligooxyalkylene)sulfonates and C₁₀-C₂₂ acylamino C₁-C₆ alkylenesulfonates, C₁₀-C₂₂ alkylbenzenesulfonates, C₁₀-C₂₂ alkylnaphthalenesulfonates, C₁₀-C₂₂ α-olefinsulfonates, C₁₀-C₂₂ α-sulfo fatty acid C₁-C₆ alkylesters, C₄-C₂₂ alkyl sulfosuccinate esters, C₄-C₂₂ alkyl(oligooxyalkylene) sulfosuccinate esters, C₄-C₂₂ acylamino C₁-C₆ alkylene sulfosuccinate esters and C₄-C₂₂ acylaminoethyl(oligooxyalkylene) sulfosuccinate esters; organophosphate surfactants selected from C₁₀-C₂₂ alkylphosphates, C₁₀-C₂₂ alkyl(oligooxyalkylene)phosphates and C₁₀-C₂₂ alkylbenzenephosphates; organophosphonate surfactants selected from C₁₀-C₂₂ alkylphosphonates and C₁₀-C₂₂ alkyl(oligooxyalkylene)phosphonates; C₁₀-C₂₂ acylsarcosinates; or carboxylate surfactants selected from C₁₀-C₂₂ alkylcarboxylates and C₁₀-C₂₂ alkyl(oligooxyalkylene)carboxylates; wherein oligooxyalkylene moieties represent from one to five oxy-C₁-C₆ alkylene moieties, preferably oxyethylene moieties, linked via ether moieties. Preferably, the upper limit to the alkyl, acyl, olefin and fatty acid chain length of C₂₂ in the preceding sentence is C₁₆. Moreover, the hydrocarbon moieties of the alkyl, acyl, alkylene, olefin and fatty acid moieties are linear or branched.

The anionic charge on such anionic moieties is countered under aqueous conditions by a countercation selected from alkali metal, C₁-C₃ alkyl ammonium, C₁-C₃ alkanol ammonium, and ammonium cations. It should be noted that C₁-C₃ alkyl ammonium, C₁-C₃ alkanol ammonium, and ammonium cations are formed upon treatment of the free-acid form of the surfactant with C₁-C₃ alkyl amine, C₁-C₃ alkanol amine, and ammonia, respectively, particularly under aqueous conditions. Preferably, the countercation is selected from alkali metal, C₁-C₃ alkylammonium, tri(C₁-C₃ alkanol)ammonium, di(C₁-C₃ alkanol)ammonium and ammonium cations. In a more preferred embodiment, the countercation is selected from sodium or triethanolammonium cations.

Thus, the C₁₀-C₂₂ or C₁₀-C₁₆ alkylsulfates are preferably selected from dodecyl sulfates, tetradecyl sulfates, cocosulfates, tallow alcohol sulfates and mono-C₁₂-C₁₈ alkylsulfates, or mixtures thereof. Preferred examples of such sulfates are selected from sodium dodecylsulfate, potassium dodecylsulfate, triethanolammonium dodecylsulfate (*i.e.* triethanolamine laurylsulfate, CAS 90583-18-9, marketed under registered trademark Texapon T42), diethanolammonium dodecylsulfate, ammonium dodecyl sulfate, sodium tetradecyl sulfate, triethanolammonium tetradecyl sulfate, sodium cocosulfates (CAS 97375-27-4), sodium tallow alcohol sulfates (CAS 68140-10-3) and sodium mono-C₁₂-C₁₈ alkylsulfates (CAS 68955-19-1).

The C₁₀-C₂₂ or C₁₀-C₁₆ alkyl(oligooxyalkylene)sulfates (also known as alkyl ether sulfates and ether sulfates) are preferably selected from laureth sulfates, trideceth sulfates, myreth sulfates and pentadeceth sulfates, or mixtures thereof such as C₁₂-C₁₄ alkyl(oligooxyalkylene) sulfates (*e.g*. CAS 68891-38-3, marketed under registered trademark Texapon N 40 IS). The laureth sulfates (*e.g*. CAS 3088-31-1, CAS 9004-82-4, CAS 27028-82-6) preferably include ethyleneglycol, diethyleneglycol and triethyleneglycol lauryl ether sulfates, or mixtures thereof. The trideceth sulfates (*e.g*. CAS 25446-78-0) preferably include ethyleneglycol, diethyleneglycol and triethyleneglycol tridecyl ether sulfates, or mixtures thereof. The myreth sulfates (*e.g*. CAS 25446-80-4) preferably include ethyleneglycol, diethyleneglycol and triethyleneglycol myristyl ether sulfates, or mixtures thereof. The pentadeceth sulfates preferably include ethyleneglycol, diethyleneglycol and triethyleneglycol pentadecyl ether sulfates, or mixtures thereof.

The C₁₀-C₂₂ alkylbenzyl(oligoxyalkylene)sulfates (also known as alkylbenzene ether sulfates and benzene ether sulfates) are preferably *p-*C₁₀-C₁₆ alkylbenzyl(oligoxyethylene)sulfates or *p-*C₁₀-C₁₆ alkylbenzyl(oligoxypropylene)sulfates selected from laurylbenzyl(oligoxyethylene)sulfates, laurylbenzyl(oligoxypropylene)sulfates, tridecylbenzyl(oligoxyethylene)sulfates, tridecylbenzyl(oligoxypropylene)sulfates, myristylbenzyl(oligoxyethylene)sulfates and myristylbenzyl(oligoxypropylene)sulfates, wherein the oligoxyethylene moieties are selected from ethyleneglycol, diethyleneglycol and triethyleneglycol moieties, or mixtures thereof.

The C₁₀-C₂₂ or C₁₀-C₁₆ alkylsulfonates (*e.g*. CAS 85711-69-9) are preferably selected from dodecylsulfonates, tetradecyl sulfonates, myristyl sulfonates, cocosulfonates, tallow alcohol sulfonates and C₁₃-C₁₇ sec-alkyl sulfonates, or mixtures thereof.

The C₁₀-C₂₂ or C₁₀-C₁₆ alkyl(oligoxyalkylene)sulfonates (also known as alkoxyalkanesulfonates) are preferably selected from laureth sulfonates, trideceth sulfonates, myreth sulfonates and pentadeceth sulfonates, or mixtures thereof. The laureth sulfonates (*e.g*. CAS 9004-82-4) include ethyleneglycol, diethyleneglycol, triethyleneglycol, propyleneglycol, dipropyleneglycol and tripropyleneglycol lauryl ether sulfonates, or mixtures thereof. The trideceth sulfonates, myreth sulfonates and pentadeceth sulfonates likewise include the corrresponding ethyleneglycol, diethyleneglycol, triethyleneglycol, propyleneglycol, dipropyleneglycol and tripropyleneglycol alkyl ether sulfonates, or mixtures thereof.

The C₁₀-C₂₂ or C₁₀-C₁₆ acyl(oligoxyalkylene)sulfonates (also known as acyloxyalkanesulfonates) are preferably selected from lauroyl ether sulfonates, tridecanoyl ether sulfonates, myristoyl ether sulfonates and pentadecanoyl ether sulfonates, or mixtures thereof. The lauroyl ether sulfonates include ethyleneglycol, diethyleneglycol, triethyleneglycol, propyleneglycol, dipropyleneglycol and tripropyleneglycol lauroyl ether sulfonates, or mixtures thereof. The tridecanoyl ether sulfonates, myristoyl ether sulfonates and pentadecanoyl ether sulfonates likewise include the corresponding ethyleneglycol, diethyleneglycol, triethyleneglycol, propyleneglycol, dipropyleneglycol and tripropyleneglycol alkanoyl ether sulfonates, or mixtures thereof.

The C₁₀-C₂₂ or C₁₀-C₁₆ acylamino C₁-C₆ alkylenesulfonates (also known as acylaminoalkanesulfonates) are preferably selected from lauroyl amide sulfonates, tridecanoyl amide sulfonates, myristoyl amide sulfonates and pentadecanoyl amide sulfonates, or mixtures thereof. The lauroyl amide sulfonates preferably include sodium methylene, ethylene, propylene, butylene or pentylene lauroyl amide sulfonates, or mixtures thereof. The tridecanoyl amide sulfonates, myristoyl amide sulfonates and pentadecanoyl amide sulfonates likewise include the corresponding methylene, ethylene, propylene, butylene or pentylene alkanoyl amide sulfonates, or mixtures thereof.

The C₁₀-C₂₂ or C₁₀-C₁₆ alkylbenzenesulfonates (also known as alkylbenzenesulfonates) are preferably selected from *p*-decylbenzenesulfonates, *p-*dodecylbenzenesulfonates, *p*-tetradecylbenzenesulfonates, and *p-*myristylbenzenesulfonates or mixtures thereof. Preferred examples of alkylbenzenesulfonates are selected from C₁₀-C₁₃ alkylbenzenesulfonates (*e.g*. CAS 85536-14-7, CAS 84989-15-1, CAS 68411-31-4), C₁₀-C₁₆ alkylbenzenesulfonates (*e.g*. CAS 68081-81-2, CAS 68584-27-0, CAS 68910-31-6), sodium dodecylbenzenesulfonate, potassium dodecylbenzenesulfonate, and isopropylammonium dodecylbenzenesulfonate, or mixtures thereof.

The C₁₀-C₂₂ or C₁₀-C₁₆ alkylnaphthalenesulfonates (also known as alkylnaphthalenesulfonates) are preferably selected from sodium dipropylnaphthalenesulfonates and sodium dibutylnaphthalenesulfonates, or mixtures thereof.

The C₁₀-C₂₂ or C₁₀-C₁₆ α-olefinsulfonates (also known as olefinsulfonates) are preferably selected from sodium C₁₄-C₁₆ α-olefinsulfonates (CAS 68439-57-6) and sodium C₁₆-C₁₈ α-olefinsulfonates, or mixtures thereof.

The C₁₀-C₂₂ or C₁₀-C₁₆ α-sulfo fatty acid C₁-C₆ alkylesters (also known as α-sulfo fatty acid esters) are preferably selected from sodium salts of α-sulfo coconut fatty acid methyl esters, α-sulfo palm kernel fatty acid methyl esters and α-sulfo tallow fatty acid methyl esters, or mixtures thereof.

The C₄-C₂₂ or C₄-C₁₆ alkyl sulfosuccinate esters are preferably selected from sulfosuccinate C₁₀-C₂₂ mono-alkyl esters and sulfosuccinate C₄-C₁₂ di-alkyl esters, or mixtures thereof. Preferably, the sulfosuccinate C₁₀-C₂₂ mono-alkyl esters are disodium lauryl sulfosuccinate or disodium myristyl sulfosuccinate. Preferably the sulfosuccinate C₅-C₁₂ di-alkyl esters are sodium 1,4-bis(2-ethylhexyl)sulfosuccinate, sodium dioctylsulfosuccinate or sodium 1,4-bis(1-methylheptyl)sulfosuccinate.

The C₄-C₂₂ or C₄-C₁₆ alkyl(oligooxyalkylene) sulfosuccinate esters are preferably selected from sulfosuccinate C₁₀-C₂₂ mono-fatty acid ethoxylate esters and sulfosuccinate C₄-C₁₂ di-fatty acid ethoxylate esters, or mixtures thereof. Preferably, the sulfosuccinate C₁₀-C₂₂ mono-fatty acid ethoxylate ester is disodium lauryltriethyleneglycol sulfosuccinate (CAS 58450-52-5). Preferably the sulfosuccinate C₄-C₁₂ di-fatty acid ethoxylate ester is and sodium dihexyltriethyleneglycol sulfosuccinate.

The C₄-C₂₂ or C₄-C₁₆ acylamino C₁-C₆ alkylene sulfosuccinate esters (also known as sulfosuccinamates) are preferably selected from sulfosuccinate C₁₀-C₂₂ mono-fatty acid ethanolamides and sulfosuccinate C₄-C₂₂ di-fatty acid ethanolamides, or mixtures thereof. Preferably, the sulfosuccinate C₁₀-C₂₂ mono-fatty acid ethanolamide is disodium lauroylaminoethyl sulfosuccinate. Preferably, the sulfosuccinate C₄-C₂₂ di-fatty acid ethanolamide is sodium bis(lauroylaminoethyl) sulfosuccinate.

The C₄-C₂₂ or C₄-C₁₆ acylaminoethyl(oligooxyalkylene) sulfosuccinate esters (also known as sulfosuccinamates) are preferably selected from sulfosuccinate C₁₀-C₂₂ mono-acylaminoethyl(oligooxyethylene)esters and sulfosuccinate C₄-C₂₂ di-acylaminoethyl(oligooxyethylene)esters, or mixtures thereof. These sulfosuccinamates include the monooxyethylene, dioxyethylene and trioxyethylene derivatives, or mixtures thereof.

The C₁₀-C₂₂ alkylphosphates are preferably selected from C₁₀-C₁₆ monoalkyl, dialkyl and trialkyl phosphates, or mixtures thereof. The or C₁₀-C₁₆ monoalkyl, dialkyl and trialkyl phosphates are preferably selected from dodecylphosphates, tetradecylphosphates, myristylphosphates, cocophosphates, tallow alcohol phosphates and C₁₃-C₁₇ sec-alkyl phosphates (CAS 85711-69-9), wherein each alkyl substituent may be the same or different, or mixtures thereof.

The C₁₀-C₂₂ alkyl(oligooxyalkylene)phosphates (also known as alkylether phosphates) are preferably selected from or C₁₀-C₁₆ mono-, di- and tri-[alkyl(oligooxyalkylene)] phosphates, or mixtures thereof. The or C₁₀-C₁₆ mono-, di- and tri-[alkyl(oligooxyalkylene)] phosphates are preferably selected from laureth phosphates, trideceth phosphates, myreth phosphates and pentadeceth phosphates), wherein each alkylether substituent may be the same or different, or mixtures thereof. The laureth phosphates (*e.g*. CAS 42612-52-2, CAS 68954-87-0, CAS 58318-92-6) include ethyleneglycol, diethyleneglycol, triethyleneglycol, propyleneglycol, dipropyleneglycol and tripropyleneglycol lauryl ether phosphates, or mixtures thereof. The trideceth phosphates, myreth phosphates and pentadeceth phosphates likewise include the corresponding ethyleneglycol, diethyleneglycol, triethyleneglycol, propyleneglycol, dipropyleneglycol and tripropyleneglycol alkylether phosphates, or mixtures thereof.

The C₁₀-C₂₂ or C₁₀-C₁₆ alkylbenzenephosphates (also known as alkylbenzenephosphates) are preferably selected from *p*-decylbenzenephosphates, *p-*dodecylbenzenephosphates, *p*-tetradecylbenzenephosphates, and *p-*myristylbenzenephosphates or mixtures thereof. Preferred examples of alkylbenzenephosphates are sodium dodecylbenzenephosphate, potassium dodecylbenzenephosphate, and isopropylammonium dodecylbenzenephosphate, or mixtures thereof.

The C₁₀-C₂₂ alkylphosphonates are preferably selected from C₁₀-C₁₆ monoalkyl, dialkyl and trialkyl phosphonates, or mixtures thereof. The or C₁₀-C₁₆ monoalkyl, dialkyl and trialkyl phosphonates are preferably selected from dodecylphosphonates, tetradecylphosphonates, myristylphosphonates, cocophosphonates, and tallow alcohol phosphonates, wherein each alkyl substituent may be the same or different, or mixtures thereof.

The C₁₀-C₂₂ alkyl(oligooxyalkylene)phosphonates (also known as alkylether phosphonates) are preferably selected from or C₁₀-C₁₆ mono-, di- and tri-[alkyl(oligooxyalkylene)] phosphonates, or mixtures thereof. The or C₁₀-C₁₆ mono-, di- and tri-[alkyl(oligooxyalkylene)] phosphonates are preferably selected from laureth phosphonates, trideceth phosphonates, myreth phosphonates and pentadeceth phosphonates), wherein each alkylether substituent may be the same or different, or mixtures thereof. The above phosphonates (*e.g*. CAS 68954-87-0) include ethyleneglycol, diethyleneglycol, triethyleneglycol, propyleneglycol, dipropyleneglycol and tripropyleneglycol lauryl ether phosphonates, or mixtures thereof.

The C₁₀-C₂₂ acylsarcosinates (also known as sarcosides) are preferably selected from condensation products of C₁₀-C₂₂ fatty acids, C₁₀-C₂₂ fatty acid halides or C₁₀-C₂₂ fatty acid anhydrides with amino acids or oligopeptides, wherein the oligopeptides are obtained by partial hydrolysis of collagen. More preferably, the sarcosides are C₁₀-C₁₆ acylsarcosinates selected from sodium lauroyl sarcosinate, potassium cocoyl hydrolysed collagen, triethanolamine cocoyl hydrolysed collagen, potassium undecenoyl hydrolysed collagen and triethanolamine abietoyl hydrolysed collagen.

The C₁₀-C₂₂ or C₁₀-C₁₆ alkylcarboxylates (also known as soaps) are preferably selected from decanoates, undecanoates, dodecanoates, tridecanoates, tetradecanoates, pentadecanoates and hexadecanoates, or mixtures thereof.

The C₁₀-C₂₂ or C₁₀-C₁₆ alkyl(oligooxyalkylene)carboxylates (also known as carboxymethylated ethoxylates) are preferably selected from decyl-, undecyl-, dodecyl-, tridecyl-, tetradecyl-, pentadecyl- and hexadecyl(oligooxyethylene)acetates, wherein the oligooxyethylene moiety preferably is a monooxyethylene, dioxyethylene or trioxyethylene, or mixtures thereof.

In a preferred embodiment, the one or more anionic surfactants is selected from alkali metal, C₁-C₃ alkylammonium, di(C₁-C₃ alkanol)ammonium, tri(C₁-C₃ alkanol)ammonium, or ammonium salts of C₁₀-C₂₂ alkylsulfates, C₁₀-C₂₂ alkyl(oligooxyalkylene)sulfates, C₄-C₂₂ alkyl sulfosuccinate esters, C₁₀-C₂₂ acylsarcosinates and C₁₀-C₂₂ alkylcarboxylates, wherein oligooxyalkylene moieties represent from one to five oxy-C₁-C₆ alkylene moieties, preferably oxyethylene moieties, linked via ether moieties.

In a further preferred embodiment, the one or more anionic surfactants are selected from alkali metal, C₁-C₃ alkylammonium, di(C₁-C₃ alkanol)ammonium, tri(C₁-C₃ alkanol)ammonium, or ammonium salts of a C₁₀-C₂₂ alkyl sulfate, a C₁₀-C₂₂ alkyl(oligooxyalkylene)sulfate, a C₄-C₂₂ alkyl sulfosuccinate ester and a C₁₀-C₂₂ acylsarcosinate, and
(i) a C₁₀-C₂₂ alkyl sulfate is more preferably selected from sodium dodecylsulfate (CAS 151-21-3), potassium dodecylsulfate (CAS 4706-78-9), triethanolammonium dodecylsulfate (CAS 90583-18-9), diethanolammonium dodecylsulfate (CAS 143-00-0), ammonium dodecyl sulfate (CAS 2235-54-3), sodium tetradecyl sulfate (CAS 1191-50-0), triethanolammonium tetradecyl sulfates (CAS 4492-78-8), sodium cocosulfates (CAS 97375-27-4), sodium tallow alcohol sulfates (CAS 68140-10-3), triethanolammonium C₁₂-C₁₄ alkylsulfates (CAS 90583-18-9) and sodium C₁₂-C₁₈ alkylsulfates (CAS 68955-19-1);
(ii) a C₁₀-C₂₂ alkyl(oligooxyalkylene)sulfate is more preferably selected from sodium laureth sulfate (CAS 3088-31-1, CAS 9004-82-4), triethanolamine laureth sulfate (CAS 27028-82-6), sodium trideceth sulfate (CAS 25446-78-0), sodium myreth sulfate (CAS 25446-80-4) and sodium C₁₂-C₁₄ alkyl(oligooxyethylene) sulfates (CAS 68891-38-3);
(iii) a C₄-C₂₂ alkyl sulfosuccinate ester is more preferably selected from disodium lauryl sulfosuccinate (CAS 13192-12-6), sodium 1,4-bis(2-ethylhexyl)sulfosuccinate (CAS 577-11-7), sodium dioctylsulfosuccinate (CAS 2373-23-1), sodium dicyclohexylsulfosuccinate (CAS 23386-52-9) and sodium 1,4-bis(1-methylheptyl)sulfosuccinate (CAS 20727-33-7); and
(iv) a C₁₀-C₂₂ acylsarcosinate is more preferably selected from sodium lauroyl sarcosinate (CAS 137-16-6), potassium cocoyl hydrolysed collagen (CAS 68920-65-0), triethanolamine cocoyl hydrolysed collagen (CAS 68952-16-9), potassium undecenoyl hydrolysed collagen (CAS 68951-92-8) and triethanolamine abietoyl hydrolysed collagen (CAS 68918-77-4).

The concentration of anionic surfactant in the ophthalmic composition is from 0.001 to 3.0 g/L. In one embodiment, the concentration of anionic surfactant in the ophthalmic composition is from 0.005 to 0.5 g/L. In another embodiment, the concentration is from 0.005 to 0.1 g/L, preferably 0.005 to 0.05 g/L.

In one embodiment, the ratio of the weight of the prostaglandin to the total weight of anionic surfactant(s) [i.e. the weight ratio of the prostaglandin to anionic surfactant(s)] in the aqueous ophthalmic composition is 1.0 or more. In a preferred embodiment, the weight ratio of the prostaglandin to anionic surfactant(s) is from 1.0 to 100.0, more preferably from 2.0 to 20.0. Note that ratios reported herein are expressed as the quotient of the ratio (*e.g*. a ratio ranging from 6:2 to 6:1 is expressed as 3.0 to 6.0).

The amount of any benzalkonium chloride or other cationic surfactants in the ophthalmic composition totals at most 0.00005 g/L, preferably 0.00001 g/L or less. It is preferred that the aqueous ophthalmic composition comprises no cationic surfactants. When the amount of benzalkonium chloride or other cationic surfactants in the aqueous ophthalmic composition is greater than the above upper limit, irritating effects on the cornea may occur, and unfavourable interactions with the anionic surfactant used in the invention may occur.

In one embodiment, the aqueous ophthalmic composition additionally comprises a hydrophilic organic solvent. The hydrophilic organic solvent is an organic solvent that exhibits a solubulity in water of at least 10 g/L, preferably at least 20 g/L, more preferably at least 50 g/L. Most preferably the hydrophilic organic solvent is completely miscible in water. Preferably, the hydrophilic organic solvent is selected from one or more linear, branched or cyclic C₁-C₆ alcohols, polyols or ketones, acetonitrile, dimethylsulfoxide, dimethylformamide, and tetrahydrofuran. In a preferred embodiment the hydrophilic organic solvent is a polyol, more preferably a C₁-C₆ alkylene glycol, a poly-C₂-C₆ alkylene glycol or a C₁-C₆ alkane triol. Preferably, the hydrophilic organic solvent is selected from ethylene glycol, 1,2-propane diol, 1,3-propane diol, glycerol, sugar alcohols such as erythritol, arabitol, mannitol, sorbitol, xylitol, polyethylene glycols such as PEG 200, PEG 400, PEG 1000, polypropyleneglycols such as PPG 200, PPG 1000, and copolymers of polyethylene glycol and polypropylene glycol such as the block copolymer Synperonic PE/L61. Such hydrophilic organic solvents may be used singly or in combination. More preferably, the hydrophilic organic solvent is selected from at least one of ethylene glycol, 1,2-propane diol, 1,3-propane diol and glycerol.

The ratio of the total volume of hydrophilic organic solvent to total volume of water which is present in the aqueous ophthalmic composition is preferably from 0.01 or less. More preferably, the volume ratio of organic solvent to water in the ophthalmic composition is from 0.005 to 0.0001, most preferably from 0.002 to 0.0001.

The aqueous ophthalmic composition of the present invention may be a buffered aqueous solution. Preferably, the aqueous ophthalmic composition is buffered so as to have a pH which is within 0.5 pH units (when measured using universal indicator paper) of the pH of tears. Preferably, the pH of the ophthalmic composition is from 6.4 to 7.0, more preferably from 6.6 to 6.8. Preferably, the buffer system deployed in the buffered aqueous solution is a phosphate buffer, more preferably a K₂HPO₄/KH₂PO₄ buffer or a Na₂HPO₄/NaH₂PO₄ buffer.

The aqueous ophthalmic composition of the present invention is preferably isoosmotic and isotonic with the fluids found in the ocular cavity, namely tears. Preferably, the osmolarity of the aqueous ophthalmic composition is from 260-315 mOsm/L. Thus, the aqueous ophthalmic composition preferably comprises one or more salts, preferably sodium chloride. The amount of such salts comprised in the aqueous ophthalmic composition is adjusted to provide a composition with an osmolarity and/or tonicity which is isoosmotic and/or isotonic with the fluids found in the ocular cavity

The aqueous ophthalmic composition of the present invention may additionally comprise one or more excipients. Preferably, such excipients are excipients which are acceptable for ophthalmic administration in that they are compatible with the tissues and fluids found in the ocular cavity. The excipients may be selected from non-ionic surfactants, antimicrobial agents, hydrophilic polymers and antioxidants.

Non-ionic surfactants are selected from ethoxylates, fatty acid esters of polyols and amine oxides. Ethoxylates (*e.g*. polyoxyl 40 stearate, Myrj 52, Brij 30, Tween 20, Tween 40, Tween 60, Tween 65, Tween 80, polyoxyl 40 hydrogenated castor oil) are preferably selected from ethoxylated, diethoxylated, triethoxylated, tetraethoxylated, pentaethoxylated or hexaethoxylated C₁₀-C₂₂ fatty acids, , C₁₀-C₂₂ fatty acid amides, C₁₀-C₂₂ fatty amides, C₁₀-C₂₂ alcohols, C₁₀-C₂₂ alkylphenols and C₁₀-C₂₂ fatty acid esters of polyols, wherein the hydrogen atom of the terminal hydroxyl group is optionally replaced with a hydrophobic moiety such as a benzyl, butyl or methyl group, or mixtures thereof. Fatty acid esters of polyols (*e.g*. sorbityl monolaurate, sorbityl monooleate, CAS 68308-54-3, CAS 128664-36-8, CAS 136797-44-9) are preferably selected from C₁₀-C₂₂ fatty acid esters of glycerol, sorbitol, sucrose and polyglucosides, or mixtures thereof. Amine oxides (*e.g*. CAS 1643-20-5, CAS 2571-88-2, CAS 61788-90-7) are preferably selected from C₁₀-C₂₂ alkyldimethylamine oxides and C₁₀-C₂₂ alkyldiethanolamine oxides. The total amounts of non-ionic surfactants in the ophthalmic composition should be lower than 5.0 g/L, preferably lower than 1.0 g/L. It may also be lower than 0.1 g/L.

Anti-microbial agents may be added to the aqueous ophthalmic composition in order to prevent microbes from growing and/or reproducing in the aqueous ophthalmic composition. The anti-microbial agents are selected from antibiotics and antifungals. Preferably the anti-microbial agent is selected from ethylene oxide, boric acid, carbapenems, cephalosporins, penicillins, quinolones, sulfonamides, tetracyclines, polyene, imidazole, triazole, thiazole, allylamines and echinocandins. The anti-microbial agent is added in an amount sufficient to halt and/or inhibit microbial growth in the aqueous ophthalmic composition for the duration of its storage prior to expiry. When the aqueous ophthalmic composition of the present invention is packaged in a single dose package, ethylene oxide is preferably used to sterilize the package prior to sealing.

Hydrophilic polymers may be added to the aqueous ophthalmic composition in order to thicken the tear film and increase corneal contact time of the prostaglandin. Preferably, the hydrophilic polymers are selected from methylcellulose, hydroxyethylcellulose, hydroxypropylmethylcellulose, polyethylene glycol, polyvinylpyrrolidone, dextran and polyvinylalcohol. The hydrophilic polymers are used in an amount sufficient to provide the aqueous ophthalmic composition with a viscosity in the range of 5 to 100 cps at 20 °C.

Antioxidants may be added to the aqueous ophthalmic composition in order to prevent oxidation of the prostaglandin. Preferably, antioxidants are selected from ascorbic acid, acetylcysteine, EDTA and sodium bisulfite.

The present invention also relates to a method for manufacture of the herein-described aqueous ophthalmic composition. The method for manufacture of the aqueous ophthalmic composition comprises dissolving the ingredients in water in a one step or multi-step procedure. In a preferred embodiment, the procedure has two or more steps.

The two-step method comprises a first step of dissolving a prostaglandin and one or more anionic surfactants in water or, preferably, in a hydrophilic organic solvent or water/ hydrophilic organic solvent mixture. Thus, the step of dissolution is performed either in the absence of water or in the presence of water. When the step of dissolution is performed in the presence of water, water may be added to the hydrophilic organic solvent as pure water, as an aqueous solution comprising the one or more anionic surfactants, as an aqueous saline solution or as an aqueous buffer solution. Dissolution is preferably perfomed by agitation of the mixture of a prostaglandin and one or more anionic surfactants in a hydrophilic organic solvent, optionally in the presence of water. Preferably agitation comprises moving the mixture in such a way that bulk displacement thereof occurs, preferably by shaking, swirling or stirring.

The two-step method additionally comprises a second step of diluting the solution obtained in the first step with water optionally including one or more ophthalmically acceptable excipients. Thus, the step of dissolution is performed by adding water to the solution obtained in the first step, or by adding an aqueous solution comprising one or more ophthalmically acceptable excipients in the presence of water. Preferably an aqueous solution comprising one or more ophthalmically acceptable excipients is an aqueous buffer solution or an aqueous saline solution.

The method of the present invention may additionally comprise further steps of sterilization and/or packaging.

The step of sterilizing is selected from a step of filtration with a microbial filter or an ultrafilter, and/or chemical sterilization by treatment with an antimicrobial agent or by displacement of oxygen from the solution. When the method for manufacture of the aqueous ophthalmic composition comprises a step of filtration, this is preferably employed as a third step, wherein the diluted solution obtained in the second step is subjected to filtration. When the method for manufacture of the aqueous ophthalmic composition comprises a step of chemical sterilization, this is effected before, during or after any of the first and second steps.

The step of packaging involves transferring the aqueous ophthalmic composition of the present invention to one or more packages. Each package is a sealable package which may be a single-dose or multiple-dose package. The package preferably comprises plastic and/or glass, wherein the plastic is a plastic selected from polyethylene, polypropylene and/or a copolymer of polyethylene and polypropylene, more preferably polyethylene. The package is selected from a one-piece package or a two-piece package comprising a base and a cap. Optionally, the package may comprise a valve, dropper, or plunger for dispensing the aqueous ophthalmic composition of the present invention. The step of packaging preferably deploys blow-fill-seal technology to form single-dose packages comprising the aqueous ophthalmic composition of the present invention. Alternatively, the step of packaging preferably involves transferring the aqueous ophthalmic composition of the present invention to one or more multiple-dose packages. When the method of the present invention involves a step of packaging in a multiple-dose package, the aqueous ophthalmic composition of the present invention preferably comprises an antimicrobial agent. Multiple-dose packages may contain a filter for preventing microbial contamination as described in EP 2 193 795 A1.

Single-dose packages may contain between 0.2 and 0.8 ml, preferably between 0.3 to 0.5 ml per package. Multi-dose packages may contain between 1 and 25 ml, preferably between 2 and 10 ml per package.

Preferably, the method for manufacture of the aqueous ophthalmic composition of the present invention is performed at between 0 and 10°C, more preferably between 3 and 8°C, most preferably at between 5 and 7°C. In one embodiment, either or both of the two steps (or any further step) of the method for manufacture of the aqueous ophthalmic composition of the present invention are performed at between 0 and 10 °C, preferably between 3 and 8 °C. Preferably, both of the two st eps of the method of manufacture are performed at between 0 and 10 °C, more preferably between 3 and 8 °C. When the method for manufacture of the aqueous ophthalmic composition of the present invention is performed at temperature as defined above, degradation of the prostaglandin can be suppressed.

Preferably, the method for manufacture of the aqueous ophthalmic composition of the present invention is also performed in the absence of oxygen under a non-oxidising atmosphere, more preferably under a nitrogen or argon atmosphere. When the method of the present invention comprises a step of packaging, this step is preferably performed under a nitrogen atmosphere.

The present invention also relates to a method of preventing or treating glaucoma or intraocular hypertension, or of providing neuroprotection to retinal tissues, comprising topical administration of the aqueous ophthalmic composition of the present invention. Topical administration of the aqueous ophthalmic composition is effected by applying said ophthalmic composition to the ocular cavity, preferably by administration to at least one of the conjunctiva, cornea, sclera and the inner surfaces of the eyelid.

Preferably, the aqueous ophthalmic composition is administered in doses of a volume and frequency sufficient to reduce the intraocular pressure. Preferably, one or a few drops are applied to the ocular cavity with each topical administration. Preferably the aqueous ophthalmic composition of the present invention is administered in such doses from 1 to 6 times daily, more preferably from 1 to 3 times daily.

### EXAMPLES

### Example 1

A latanoprost formulation of batch size 7.5 liters was prepared from the following components:

| Ingredient | Quantity |
|---|---|
| latanoprost | 0.375 g |
| disodium phosphate | 35.55 g |
| monosodium phosphate monohydrate | 34.5 g |
| TEXAPON™ N 40 IS | 0.55 g |
| propylene glycol | 5 ml |
| sodium chloride | 30.75 g |
| 1.0 M NaOH or diluted phosphoric acid | as required for final pH 6.6-6.8 |
| water for injection | for final volume of 7.5 l |

TEXAPON™ N 40 IS was obtained from Cognis. It is a solution containing 27% sodium lauryl ether sulphate. The propylene glycol used in this and the following examples is propane-1,2-diol.

First, a latanoprost solution was made as follows. Latanoprost, sodium lauryl ether sulfate and propylene glycol were mixed in the amounts indicated in the above table in a glass vessel. The vessel was shaken gently while cooling on a water/ice mixture until the components dissolved, which takes about 5 minutes. Then, 5 ml of water were added and shaken gently for another 5 minutes, while cooling was continued). Then, 10 ml of water were added and shaken gently for 10 minutes during which time cooling was continued.

In a separate vessel, the disodium phosphate, the monosodium phosphate monohydrate and the sodium chloride was dissolved in about 6 l of water (80% of the total volume of the batch size). When the solids were dissolved, the latanoprost solution made as described in the preceding paragraph was added, followed by gentle shaking for 10 minutes. Then, the pH was set to be within the range of 6.6-6.8 with the 1.0 M sodium hydroxide solution or the diluted phosphoric acid as necessary. Next, water was added up to the final volume of 7.5 l.

The obtained mixture was filtered through a Milliflex™-100 equipment using a 0.2 µm membrane. The filtrate was dispensed into 2.5 ml PE bottles in a bottlepack system, and used in the tests of the following example 2. The bottlepack system allows filling of the bottles under sterile conditions under laminar air flow. The bottles were filled using the blow-fill-seal methodology under nitrogen as an inert gas.

### Example 2

The latanoprost formulation of Example 1 was tested on its content of latanoprost and degradation products of latanoprost:
- before and after the filtration through the 0.2 µm membrane of Example 1;
- in a short stability test.

The Milliflex™-100 filter system mentioned above was used for filtration. The microbiology test was made according to the European Pharmacopeia. In the appearance test, any change in the solution such as color or turbidity was checked visually.

The results of 3 independent laboratory batches before and after filtration are as follows:

| Batch LTX-01 | | |
|---|---|---|
| Parameter | Before filtration | After filtration |
| Appearance | Complies | Complies |
| pH | 6.69 | 6.62 |
| Latanoprost assay (µg/ml) | 50.33 | 50.19 |
| Sum of all degradation products | 0.058% | 0.063% |
| Microbiological control | < 10 CFU/100 ml | sterile |

| Batch LTX-02 | | |
|---|---|---|
| Parameter | Before filtration | After filtration |
| Appearance | Complies | Complies |
| pH | 6.72 | 6.72 |
| Latanoprost assay (µg/ml) | 49.96 | 49.88 |
| Sum of all degradation products | 0.071 % | 0.073% |
| Microbiological control | < 10 CFU/100 ml | sterile |

| Batch LTX-03 | | |
|---|---|---|
| Parameter | Before filtration | After filtration |
| Appearance | Complies | Complies |
| pH | 6.78 | 6.78 |
| Latanoprost assay (µg/ml) | 50.04 | 49.89 |
| Sum of all degradation products | 0.053% | 0.051 % |
| Microbiological control | < 10 CFU/100 ml | sterile |

The above data show that essentially no latanoprost was lost in the filtration step.

The stability test was conducted on 3 separate batches of formulations. The formulations were stored at a temperature of 5-8°C for 30 days at a relative humidity of 60% in sterilised 50 ml glass bottles. The results from 3 separate tests are as follows.

| Batch LTX-01 | | |
|---|---|---|
| Parameter | Time 0 | Time 30 days |
| Appearance | Complies | Complies |
| pH | 6.62 | 6.63 |
| Latanoprost assay (µg/ml) | 50.19 | 50.18 |
| Sum of all degradation products | 0.063% | 0.061% |
| Microbiological control | < 10 CFU/100 ml | sterile |

| Batch LTX-02 | | |
|---|---|---|
| Parameter | Time 0 | Time 30 days |
| Appearance | Complies | Complies |
| pH | 6.72 | 6.73 |
| Latanoprost assay (µg/ml) | 49.88 | 49.79 |
| Sum of all degradation products | 0.073% | 0.069% |
| Microbiological control | < 10 CFU/100 ml | Sterile |

| Batch LTX-03 | | |
|---|---|---|
| Parameter | Time 0 | Time 30 days |
| Appearance | Complies | Complies |
| pH | 6.78 | 6.77 |
| Latanoprost assay (µg/ml) | 49.89 | 49.92 |
| Sum of all degradation products | 0.051 % | 0.053% |
| Microbiological control | < 10 CFU/100 ml | Sterile |

The data from the above tables demonstrate that there was no noticeable loss of latanoprost under the conditions used.

### Example 3

Latanoprost formulations with alternative anionic surfactants. Batch size: 7.5 liters

| Ingredients | Quantity |
|---|---|
| latanoprost | 0.375 g |
| disodium phosphate | 35.55 g |
| monosodium phosphate monohydrate | 34.5 g |
| anionic surfactant: | 0.150 g |
| sodium lauryl ether sulfate or sodium lauroyl sarcosinate or triethanolamine lauryl ether sullfate | |
| propylene glycol | 5 ml |
| sodium chloride | 30.75 g |
| 1.0 M NaOH or diluted phosphoric acid | as required for final pH 6.6-6.8 |
| water for injection | for final volume of 7.5 l |

For each of the 3 anionic surfactants listed in the above table, separate latanoprost formulations were made following the procedure described in Example 1 using the quantities of components given in the above table. Triethanolamine lauryl sulfate was the commercial product TEXAPON™ T42. The amounts given for the surfactants are those of the indicated chemical substance. The 3 formulations can be tested as described in Example 2.

| Batch LTX 05 - Lauryl ether sulfat sodium | |
|---|---|
| Parameter | Time 0 |
| Appearance | Complies |
| pH | 6.64 |
| Latanoprost assay (µg/ml) | 50.44 |
| Sum of all degradation products | 0.015% |
| Microbiological control | < 10 CFU/100 ml |

| Batch LTX-06 - Lauroyl sarcosinate sodium | |
|---|---|
| Parameter | Time 0 |
| Appearance | Complies |
| pH | 6.69 |
| Latanoprost assay (µg/ml) | 50.14 |
| Sum of all degradation products | 0.019% |
| Microbiological control | < 10 CFU/100 ml |

| Batch LTX-07 - Lauryl ether triethanolamine | |
|---|---|
| Parameter | Time 0 |
| Appearance | Complies |
| pH | 6.70 |
| Latanoprost assay (µg/ml) | 50.12 |
| Sum of all degradation products | 0.018% |
| Microbiological control | < 10 CFU/100 ml |

### Example 4

Latanoprost formulations containing different concentrations of anionic surfactants. Formulation for 7.5 liters.

| Ingredients | Quantity |
|---|---|
| latanoprost | 0.375 g |
| disodium phosphate | 35.55 g |
| monosodium phosphate monohydrate | 34.5 g |
| anionic surfactant: | |
| sodium lauryl ether sulfate | 0.150 g |
| or | 0.300 g |
| or | 0.450 g |
| propylene glycol | 5 ml |
| sodium chloride | 30.75 g |
| 1.0 M NaOH or diluted phosphoric acid water for injection | as required for final pH 6.6-6.8 for final volume of 7.5 l |

For each of the 3 anionic surfactant concentrations of sodium lauryl ether sulfate listed in the above table, a separate latanoprost formulation was made following the procedure described in Example 1. The 3 formulations can be tested as described in Example 2.

The times required for dissolution in the first dissolution step wherein latanoprost, the sodium lauryl ether sulfate) and propylene glycol are mixed in a glass vessel as described in Example 1 were measured:

| Batch | Time for dissolution of latanoprost |
|---|---|
| Batch LTX 08 with 0.150 g surfactant | < 5 minutes |
| Batch LTX 09 with 0.300 g surfactant | < 5 minutes |
| Batch LTX 010 with 0.450 g surfactant | < 5 minutes |

| Batch LTX 08 - Lauryl ether sulfat sodium 150 mg | |
|---|---|
| Parameter | Time 0 |
| Appearance | Complies |
| pH | 6.72 |
| Latanoprost assay (µg/ml) | 50.31 |
| Sum of all degradation products | 0.019% |
| Microbiological control | < 10 CFU/100 ml |

| Parameter | Time 0 |
|---|---|
| Appearance | Complies |
| pH | 6.72 |
| Latanoprost assay (µg/ml) | 50.26 |
| Sum of all degradation products | 0.021 % |
| Microbiological control | < 10 CFU/100 ml |
| Appearance | Complies |
| pH | 6.69 |
| Latanoprost assay (µg/ml) | 50.28 |
| Sum of all degradation products | 0.015% |
| Microbiological control | < 10 CFU/100 ml |

### Example 5

Comparative tests on the speed of dissolution of latanoprost with different surfactants. Formulation for batch size of 7.5 liters

| Ingredients | Quantity |
|---|---|
| latanoprost | 0.375 g |
| disodium phosphate | 35.55 g |
| monosodium phosphate monohydrate | 34.5 g |
| surfactant: | 0.300 g |
| sodium lauryl ether sulfate | |
| or Cremophor RH40 | |
| or Polysorbate 80 | |
| propylene glycol | 5 ml |
| sodium chloride | 30.75 g |
| 1.0 M NaOH or diluted phosphoric acid | as required for final pH 6.6-6.8 |
| water for injection | for final volume of 7.5 l |

For each of the 3 surfactants listed in the above table, a separate latanoprost formulation was made using the same amount of the surfactants indicated in the above table following the procedure described in Example 1.

The times required for dissolution in the first dissolution step wherein latanoprost, the surfactant and propylene glycol are mixed in a glass vessel as described in Example 1 were measured. The results are as follows:

| Batch | Time for dissolution of latanoprost |
|---|---|
| Batch LTX 011 with sodium lauryl ether sulfate | < 5 minutes |
| Batch LTX 12 with Cremophor RH40 | 23 minutes |
| Batch LTX 013 with Polysorbate 80 | 37 minutes |

At 0°C, Cremophor RH40 and Polysorbate 80 are not liquid. The presence of a creamy substance was observed until all the material was dissolved.

## Claims

1. An aqueous ophthalmic composition comprising
(i) a prostaglandin; and
(ii) one or more anionic surfactants,
**characterized in that**
the concentration of anionic surfactant in the ophthalmic composition is from 0.001 to 3.0 g/L, preferably 0.005 to 0.5 g/L, more preferably 0.005 to 0.1 g/L.

2. The aqueous ophthalmic composition according to claim 1, wherein the prostaglandin is of the following formula (1): 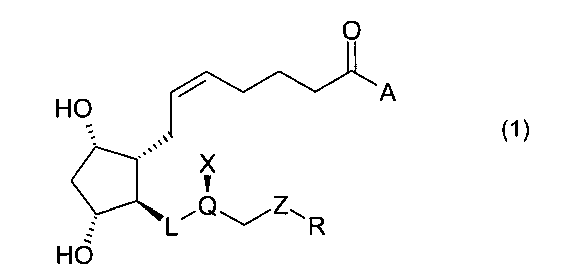 wherein
A is a linear or branched C₁-C₆ alkoxy moiety or a linear or branched mono- or di-C₁-C₆ alkylamino moiety;
L is a (CH₂)₂ moiety or a *trans*-CH=CH moiety;
A is C, a CH moiety, or a CF moiety, with the proviso that when
(a) Q is C, X is O and the Q-X bond is a double bond,
(b) Q is CH, X is OH, and
(c) Q is CF, X is F;
R is a linear, branched or cyclic C₃-C₈ alkyl moiety, a phenyl moiety, or a phenyl moiety which is substituted with
(i) a linear or branched C₁-C₃ alkyl moiety,
(ii) a halogen, or
(iii) a linear or branched C₁-C₃ haloalkyl moiety; and
Z is O or a CH₂ moiety.

3. The aqueous ophthalmic composition according to claims 1 or 2, wherein the prostaglandin is a prostaglandin FP receptor agonist.

4. The aqueous ophthalmic composition according to any of claims 1 to 3, wherein the weight ratio of the prostaglandin to the anionic surfactant(s) in said composition is 1.0 or more.

5. The aqueous ophthalmic composition according to any of claims 1 to 4, wherein the amount of benzalkonium chloride or other cationic surfactants in the ophthalmic composition totals 0.00005 g/L or less, preferably 0.00001 g/L or less.

6. The aqueous ophthalmic composition according to any of claims 1 to 5, wherein the one or more anionic surfactants are alkali metal, C₁-C₃ alkylammonium, di(C₁-C₃ alkanol)ammonium, tri(C₁-C₃ alkanol)ammonium, or ammonium salts of C₁₀-C₂₂ alkylsulfates, C₁₀-C₂₂ alkyl(oligooxyalkylene)sulfates, C₄-C₂₂ alkyl sulfosuccinate esters, C₁₀-C₂₂ acylsarcosinates and C₁₀-C₂₂ alkylcarboxylates, wherein oligooxyalkylene moieties represent from one to five oxy-C₁-C₆ alkylene moieties, preferably oxyethylene moieties, linked via ether moieties.

7. The aqueous ophthalmic composition according to any of claims 1 to 6, wherein the one or more anionic surfactants are selected from alkali metal, C₁-C₃ alkylammonium, di(C₁-C₃ alkanol)ammonium, tri(C₁-C₃ alkanol)ammonium, or ammonium salts of
(i) a C₁₀-C₂₂ alkyl sulfate preferably selected from sodium dodecylsulfate, potassium dodecylsulfate, triethanolammonium dodecylsulfate, diethanolammonium dodecylsulfate, ammonium dodecyl sulfate, sodium tetradecyl sulfate, triethanolammonium tetradecyl sulfates, sodium cocosulfates, sodium tallow alcohol sulfates, triethanolammonium C₁₂-C₁₄ alkylsulfates and sodium C₁₂-C₁₈ alkylsulfates;
(ii) a C₁₀-C₂₂ alkyl(oligooxyalkylene)sulfate preferably selected from sodium laureth sulfate, triethanolamine laureth sulfate, sodium trideceth sulfate, sodium myreth sulfate and sodium C₁₂-C₁₄ alkyl(oligooxyethylene) sulfates;
(iii) a C₄-C₂₂ alkyl sulfosuccinate ester preferably selected from disodium lauryl sulfosuccinate, sodium 1,4-bis(2-ethylhexyl)sulfosuccinate, sodium dioctylsulfosuccinate, sodium dicyclohexylsulfosuccinate and sodium 1,4-bis(1-methylheptyl)sulfosuccinate; and
(iv) a C₁₀-C₂₂ acylsarcosinate preferably selected from sodium lauroyl sarcosinate, potassium cocoyl hydrolysed collagen, triethanolamine cocoyl hydrolysed collagen, potassium undecenoyl hydrolysed collagen and triethanolamine abietoyl hydrolysed collagen.

8. The aqueous ophthalmic composition according to any of claims 1 to 7, wherein the prostaglandin is selected from latanoprost, bimatoprost, travoprost, tafluprost and unoprostone isopropyl.

9. The aqueous ophthalmic composition according to any of claims 1 to 8, wherein the ophthalmic composition additionally comprises a hydrophilic organic solvent, preferably a polyol, more preferably a C₁-C₆ alkylene glycol, a poly-C₂-C₆ alkylene glycol or a C₁-C₆ alkane triol.

10. A method for manufacture of an aqueous ophthalmic composition according to any of claims 1 to 9, which comprises the following steps:
(i) dissolving a prostaglandin and one or more anionic surfactants in a hydrophilic organic solvent, optionally in the presence of water; and
(ii) diluting the solution obtained in step (i) with water optionally including one or more ophthalmically acceptable excipients.

11. The method according to claim 10, wherein steps (i) and/or (ii) are performed at between 0 and 10°C, preferably at between 3 and 8°C.

12. A method of preventing or treating glaucoma or intraocular hypertension, or of providing neuroprotection to retinal tissues, comprising topical administration of the aqueous ophthalmic composition according to any of claims 1 to 9 to an affected eye.

13. A packaged prostaglandin product, comprising the aqueous ophthalmic composition of any one of claims 1 to 9 sealed in a plastic container.

14. The packaged prostaglandin product according to claim 13, wherein the plastic container is a polyethylene or polypropylene container.
